# EUROPEAN PATENT APPLICATION

(11) **EP 4 379 035 A1**
(43) Date of publication of application: **05.06.2024**
(21) Application number: 22383158.7
(22) Date of filing: 30.11.2022
(51) Int. Cl.: C12M 1/00, C12M 1/42, C12M 1/32

(54) **CELL CULTURE SYSTEM AND CELL CULTURE METHOD**

(71) Applicant: Fundació Institut de Bioenginyeria de Catalunya (IBEC), 08028 Barcelona (ES); Institució Catalana de Recerca I Estudis Avançats, 08010 Barcelona (ES); Fundació Institut Hospital del Mar d'Investigacions, 08003 Barcelona (ES)
(72) Inventor: Labernadie, Anna, 46005 Valencia (ES); Trepat Guixer, Xavier, 08014 Barcelona (ES); Calon, Alexander, 08903 Hospitalet de Llobregat (ES)
(74) Representative: Clarke, Modet y Cía., S.L.

(57) **Abstract**

The present invention is related to the field of cell biology and biomedicine. The present application relates to a cell culture system (1) comprising a body of the device (2) separating a first zone (3) and a second zone (4) and comprising at least one microchannel (5) fluidly communicating the first zone (3) with the second zone (4). It also relates to a cell culture plate or a well plate (18) comprising at least one cell culture system (1). The present invention further discloses a method for cell culturing using the cell culture system (1), comprising seeding a first cell type in the first zone (3) and seeding a second cell type on top of the first cell type. This method may further comprise the application of a treatment in the second zone (4) so that it passes to the first zone (3) through the at least one microchannel (5).

## Description

### FIELD OF THE INVENTION

The present invention is related to the field of cell biology and biomedicine, more precisely to cell culture systems and cell culture methods which allow to effectively recapitulate the organisation and composition of the stroma, including fibroblast and fibroblast-derived extracellular matrix found at the interface of solid tumours and around tissues and organs.

### BACKGROUND ART

Immunotherapy considerably improved cancer treatment and yielded durable therapeutic responses in many cancer subtypes¹. The presence of tumour-infiltrating lymphocytes (TILs) plays a determinant role in cancer progression², and abundant infiltration of Natural Killer (NK) cells correlates with better patient outcomes³. Yet, only 20 to 40 % of patients respond to immune-modulating therapies⁴. Growing evidence underscores the fundamental role of the tumour microenvironment (TME) in enhancing immunosuppressive signalling and interfering with cytotoxic immune cell infiltration in the tumour mass⁵.

The TME is a complex array of cellular and non-cellular components that include cancer-associated fibroblasts (CAFs), blood vessels, immune cells, and the extracellular matrix (ECM) interacting chemically and physically with the cancer cells. CAFs are the most abundant cell type populating the TME and support cancer cell survival as well as metastatic dissemination^{6, 7}. CAFs can acquire a pro-inflammatory phenotype characterised by the secretion of immunomodulatory molecules and chemokines (e.g. TGF-β, PD-L1) promoting an immunosuppressive environment8. In addition, CAFs are major producers and remodelers of the tumour stroma. These cells produce large quantities of collagens and fibronectins that organise into a highly dense meshwork constituting the ECM⁹.

The density, alignment, and composition of the TME surrounding the tumour can dictate favourable local regions to initiate tumour invasion and also hinder immune cells and molecules from infiltrating the tumour, thus limiting the efficacy of cancer therapies^{10, 11, 12}.

The high density of collagens in the tumour surrounding has been shown to reduce the ability of T cells to penetrate the tumour, thus supporting the notion of stromal cells acting as a physical barrier against immune infiltration^{13, 14}. Yet, how the TME composition and organisation impact immune cell trafficking in the tumour remains largely unexplored.

The development of bioengineered models enabled breakthroughs in unveiling how the physical properties of the TME, including matrix architecture, stiffness, and mechanical plasticity, impact cell motility^{7, 15, 16, 17}. Important technical and conceptual efforts are currently being made to improve the three-dimensional (3D) models for preclinical drug testing or for basic research purposes¹⁸. Despite increasing advances, the extensive use of collagen originating from rat tails, Matrigel from mouse sarcoma cells or hydrogels made from synthetic polymers strongly reduce the physiological relevance of these assays¹⁹.

Recent advances in microengineering for biology enabled the development of microfluidic platforms combined with microscopy. Those models have allowed the recreation of complex organisations of cells found within tissue and organs²⁰. In oncology, those approaches led to a better understanding of tumour angiogenesis^{21, 22}, and metastasis²³ both processes during which cell confinement and multicellular arrangement play a critical role. More recently, the use of microfluidic models enables monitoring of cancer-immune cell interaction and immunotherapy efficiencies^{24, 25, 26, 27}. Despite considerable efforts, on-chip models often require the use of scaffolding matrices and do not fully recapitulate the cellular composition, ECM deposition and more generally, the tumour architecture observed in patient solid tumours¹⁹. Technically, the 3D live imaging in those devices requires optimal optical settings to allow fast z-scanning, reduced phototoxicity and precisely capture scattered and fast cellular events such as immune cell migration^{28, 29, 30}. Yet, such approaches remain challenging using standard imaging laboratory equipment and are hardly compatible with drug screening assays.

In addition, in the state of the art, in most of the pre-existing in vitro approaches, multicellular culture is spatially compartmentalized using connected channels and/or using hydrogels. The small volume of culture media, cell suspension and hydrogels in those devices usually require skilled users to avoid technical issues such as drying or clotting of cells in microchannel and are rarely compatible with cell culture routine. The complexity of production and use of those devices still limits their compatibility with standard cell culture equipment and microscopy, consumables and high throughput screening methods.

Hence, none of the solutions available in the state of the art provides for a simple and effective way to recapitulate and reproduce the interface between a tissue/organ and the stroma (for example, the tumour margin and TME).

Notably, the interface between tissues/organs and the stroma (tissue/organ margins) is not only relevant in the case of tumours but also in many other instances, such as, for example, inflammatory disease (providing information on immune cell infiltration and treatments). Hence, in all those instances proper modelling of the *in vivo* structure is required and desirable.

Therefore, in the state of the art, there is the need for simple and easy cell culture systems and methods which allow for the recapitulation of the *in vivo* structure of the interface of tissues and organs with stroma, this is, tissues and organs margins.

The mechanical environment of cells strongly influences fundamental cellular processes such as cell fate, proliferation differentiation and migratory strategies. Over the past years, tissue engineering has put effort into producing 3D cellular environments with controlled mechanical properties and defined stiffnesses.

Document EP33378961 B1 discloses an in vitro tumour relapse assay method, which method comprises providing a 3-dimensional cell culture or tissue comprising immortalized, malignant, cancerous and/or neoplastic cells, exposing the 3-dimensional cell culture or tissue to at least one anti-cancer agent, interrupting the exposure and determining the effect of said exposure and interruption on the 3-dimensional cell culture or tissue.

Document CN108060132 A discloses a 3D co-culture model based on a tumour cell and a tumour-associated fibroblast. The tumour cell is labelled with a green fluorescent protein, the tumour-associated fibroblast is labelled with a red fluorescent protein, and the two cells and a prepared methylcellulose gel medium are used to construct the 3D co-culture model. The 3D model prepared in the invention simulates the anaerobic environment of in-vivo tumours, and the tumour cell and the tumour-associated fibroblast are co-cultured to construct the mutual promotion effect of the two cells in the in-vivo tumour microenvironment. The 3D co-culture model formed by the tumour cell and the tumour-associated fibroblast simulates the anaerobic environment of the in-vivo tumour, provides a similar effect to the in-vivo tumour microenvironment and has application prospects in basic research and clinical drugs.

Document WO2020254660 A1 discloses a device for the culture of cells, which device is able to support and/or maintain the cells within an environment which mimics one or more in vivo environmental condition(s). Using these devices, cells can be cultured or maintained under conditions which ensure that the cells behave and respond substantially as they would in vivo. Further, the cells can be stimulated or exposed to exogenous agents (drugs and the like) and any response determined to be one which is indicative of an in vivo response.

Document US11059041 B2 discloses an in vitro microfluidic "organ-on-chip" that mimics the structure and at least one function of specific areas of the epithelial system in vivo. In particular, a multicellular, layered, microfluidic culture is described, allowing for interactions between lamina propria-derived cells and the associated tissue-specific epithelial cells and endothelial cells. This in vitro microfluidic system can be used for modelling inflammatory tissue, e.g., autoimmune disorders involving epithelial and diseases involving epithelial layers. These multicellular, layered microfluidic "organ-on-chip", e.g. "epithelia-on-chip" further allow for comparisons between types of epithelial tissues, e.g., lung (Lung-On-Chip), bronchial (Airway-On-Chip), skin (Skin-On-Chip), cervix (Cervix-On-Chip), blood-brain barrier (BBB-On-Chip), etc., in additional to neurovascular tissue, (Brain-On-Chip), and between different disease states of tissue, i.e. healthy, pre-disease and diseased areas. Additionally, these microfluidic "organ-on-chips" allow the identification of cells and cell-derived factors driving disease states in addition to drug testing for reducing inflammation affecting epithelial regions.

The present invention differs from these state-of-the-art documents in the sense that none of them focuses on or recapitulates the interface/barrier between stroma and tumour, organs or tissues.

In most of the pre-existing in vitro approaches, multicellular culture is spatially compartmentalized using connected channels and/or using hydrogels. The small volume of culture media, cell suspension and hydrogels in those devices usually required a skilled user to avoid technical issues such as drying or clotting of cells in microchannel and are rarely compatible with cell culture routine.

### SUMMARY OF INVENTION

The present invention relates to a cell culture system (1) comprising:
- a body of the device (2) separating a first zone (3) and a second zone (4),
said body of the device (2) comprises at least one microchannel (5) fluidly communicating the first zone (3) with the second zone (4) and the at least one microchannel (5) comprises a first communicating end (6) in communication with the first zone (3) and a second communicating end (7) in communication with the second zone (4).

In one embodiment the body of the device (2) surrounds the first zone (3), and the second zone (4) surrounds the body of the device (2).

In one embodiment the body of the device (2) is made of a biocompatible material such as synthetic polymer, biocompatible natural polymer, glass or metal.

The present invention also relates to a method of producing the cell culture system (1) characterized in by comprising the following steps:
- Producing the body of the device (2) by mixing polydimethylsiloxane PDMS with a curing agent in a 10:1 weight-to weight ratio;
- Pouring a mixture into a mould comprising at least one microchannel (5) to form a body of the device (2);
- Curing the body of the device (2) for a temperature between 55 and 85°C and a time between 1 and 12 hours;
- Peel the body of the device (2) from the mould;
- Cut the body of the device (2) to form the first zone (3) and the second zone (4);
- Activating the body of the device (2) and the glass/plastic bottom surface (14) with plasma to enable the sealing of the body of the device (2) with a surface (14).

The present invention also relates to a cell culture plate or a well plate (18) comprising at least one cell culture system (1).

The present invention also relates to a method for cell culturing using the cell culture system (1) comprising the following steps:
a) Providing a cell culture system (1);
b) Seeding a first cell type in the inlet chamber (15) of the first zone (3);
c) Seeding a second cell type on top of the first cell type in the first zone (3).

In one embodiment the first cell type is fibroblast.

In one embodiment the second cell type are cancer cells or epithelial cells.

In one embodiment method further comprises a step d) after step c), wherein step d) is the application of a treatment in the outlet chamber (16) of the second zone (4).

In one embodiment the treatment is made with immunological cells and/or at least one drug.

In one embodiment the time between step b) and step c) is up to 4 days.

In one embodiment the time between step c) and step d) is up to 2 days.

### GENERAL DESCRIPTION

The present invention provides a solution for appropriate spatial and temporal compartmentalization of multiple cell types allowing, thus, to correctly reproduce the *in vivo* structure of the interface of tissues and organs with stroma, this is, tissues and organs margins. Therefore, the present invention can be applied, among others, to study cellular interaction and migration and the effect of drug treatment on multicellular behaviour in the tissue or organ/stroma interface.

The present invention aims to provide a novel multicellular drug screening platform to assess the impact of the tumour, tissue or organ /stroma margin (composition, biomechanical properties and organization) on immune infiltration rate upon immune-related treatment (e.i. immunotherapy, immunomodulators, engineering immune cells such as CAR T-cells, CAR NK-cells) by using MIRO - Micro Immune Response On-chip, an *in vitro* model that recapitulates the Tumour/stroma or tissue/stroma interface and assesses immune invasion.

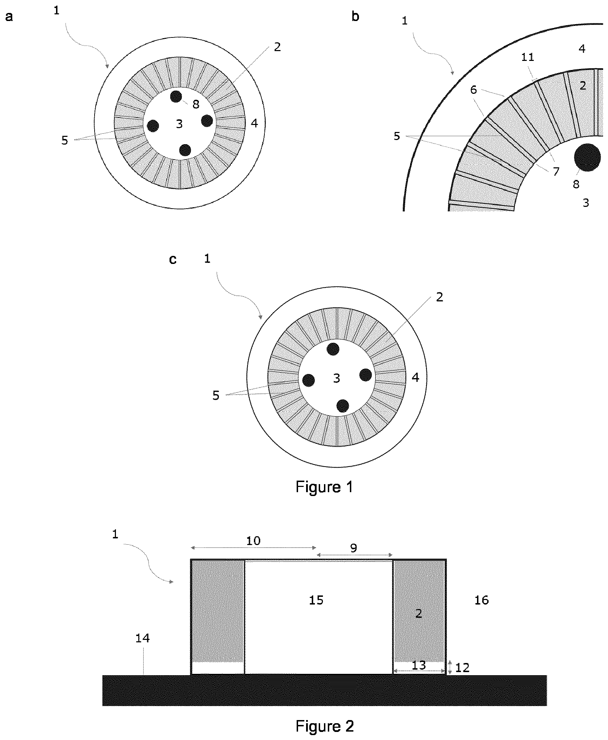

The present invention discloses a cell culture system (1) MIRO that consists of a silicon-elastomer engraved at the bottom with micron-sized channels (Figure 1). This device can be sealed by plasma treatment to commercial biocompatible cell culture Petri dishes (from single petri dishes to multi-well plate glass or plastic bottom plate). The chip presents a central inlet chamber (15) (variable size available) connected at its bottom to the outlet chamber (16) (the well of the dish) by radial repetitions of micron-sized channels. Fibroblasts seeded in suspension are allowed to attach and grow to the bottom of the dishes and to colonize for 3 days the inlet chamber (15) and the channels of the device and secreted ECM.

The present invention consists in an ex vivo platform that recapitulates the tumour/tissue microenvironment in a microfluidic device. In MIRO, cells are self-organizing a tumour/stroma or tissue/stroma interface where fibroblasts, ECM and immune cells spatially distribute in architectures reminiscent of patient tumours or tissue/stroma boundaries. Dynamic imaging of immune cell trafficking in MIRO revealed that the presence of a stromal barrier plays a critical role in immune cell guidance and tumour exclusion.

When reproducing the tumour or tissue margin (tumour/stroma tissue or organ/stroma interface) the present invention relies on the secretory capacity of the fibroblast (main producer of ECM in physiological and pathological context) to produce the ECM in the device, and the local deposition of ECM by said fibroblasts at the tumour margin presents similar features when compared with human tumour/tissue margin observed *in vivo.*

The present invention, when reproducing the tumour margin (tumour/stroma interface), provides for a short-term coculture of tumour spheroids and CAFs at high density and leads to a CAF/ECM self-generated barrier that hinders immune infiltration reducing treatment effectiveness mediated by immune cells in a similar way as observed *in vivo.* The present invention, therefore, is able to obtain reproducible tumour/stroma 3D interface surrounding the tumour core.

In addition, as noted in the examples included below, the present invention allows the generation of tumour/stroma interfaces with an abundant meshwork of ECM proteins secreted by CAF that share molecular similarities with the TME observed in patient tumour samples such as: the abundance of Fibronectin, collagen IV fibers and similar orientation along the tumour edge at the vicinity of the tumour/stroma interface.

The structures generated with the present invention enable to assess the migration pattern of immune cells, the favourable or unfavourable interaction with CAF and ECM that eventually drive immune cell entry into the tumour core in an effective and close to real way.

Compared to classical 3D environments, the present invention enables the use of simple epifluorescent to high-resolution and confocal microscopes and enables in a one-plane or small z-stack acquisition to track immune cells trajectories at the tumour margin.

The interface provided by the present invention enables to overcome some prior art limitations as there is the possibility to seal the barrier of the cell culture system (1) of the present invention onto synthetic gels with tuneable stiffnesses as substrate with physiological and pathological rigidities of the stroma. This variation of tissue stiffness provides a relevant method to evaluate the impact of the substrate.

The cell culture system (1) of the present invention has been designed to be able to work with standard volumes of media and cellular suspension to avoid periodically refilling or the use of a humid chamber.

Moreover, its shape and its tuneable size also allow and ensure the full compatibility of the cell culture system (1) of the present invention with multi-well plate with, for example, 12, 24 or 48 well plate, or multidevice per well, preferably with up to 4 mm to 14 mm diameter single petri dishes, enabling multiple drug screening for example.

Therefore, the present invention allows for the recapitulation of *in vivo* traits of the stromal barrier, i.e., the interface tissue or organ/stroma, by means of a short-term coculture strategy and a simple cell culture system (1).

As a future perspective, the use of MIRO combined with tissue engineering will be broadened to assess immune infiltration in physiological and pathological context that involve tissue or organ/stroma boundaries.

### BRIEF DESCRIPTION OF DRAWINGS

For an easier understanding of this application, figures are attached in the annexe that represents the preferred forms of implementation which nevertheless are not intended to limit the technique disclosed herein.
**Figure 1a****.** Shows an upper view of a cell culture system of the present invention.
**Figure 1b****.** Shows an upper view closer detail of a cell culture system of the present invention.
**Figure 1c****.** Shows an upper view of a cell culture system of the present invention.
**Figure 2****.** Shows a cross-section of a cell culture system of the present invention.
**Figure 3****.** Shows a cell culture plate with 12 wells, each of them with a cell culture system of the present invention.
**Figure 4****.** Shows an overview or flowchart of the method of the present invention.
**Figure 5a****, left.** Shows a representative image of MIRO co-culture on day 10. Scale bar: 2 mm.

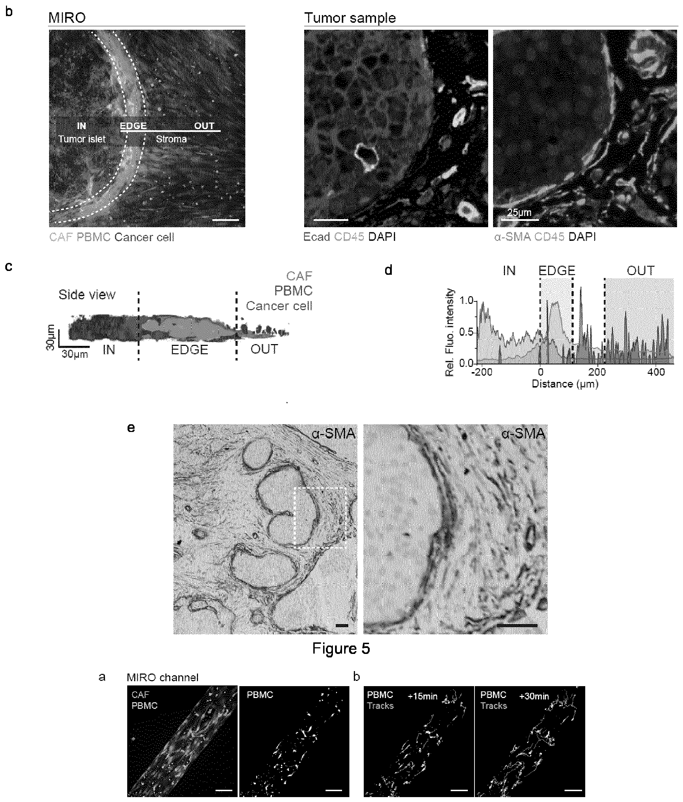

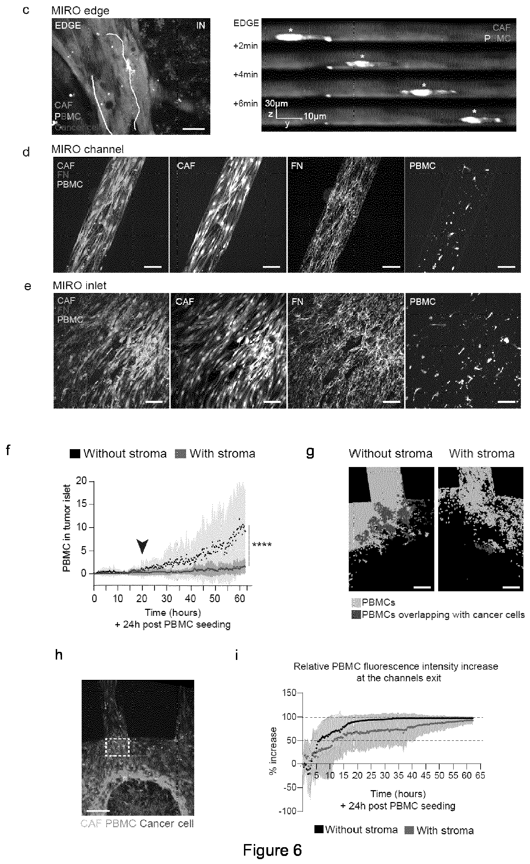

**Figure 5a****, right.** Shows a representative anti-E Cad and anti-CD45 immunofluorescence (left) vs anti-VIM and anti-CD45 immunofluorescence (right) in 4µm tissue sections from a BC patient biopsy. Scale bar: 100 µm.
**Figure 5b****, left.** Shows a representative image of the cellular organization at the tumour/stroma barrier. Scale bar: 100 µm.
**Figure 5b****, right.** Shows a representative anti-E Cad and anti-CD45 immunofluorescence (left) vs anti-VIM and anti-CD45 immunofluorescence (right) in 4µm tissue sections from a BC patient biopsy. Scale bar: 100 µm.
**Figure 5c****.** Shows a cellular distribution at the tumour/stroma boundary - 3D rendering of the tumour/stroma boundary reconstituted from z-stack confocal images CAFs, tumour spheroid PBMC. Scale bars: y-axis: 30 µm, z-axis: 30 µm.
**Figure 5d****.** Shows a cellular distribution at the tumour/stroma boundary - average fluorescence intensity profile of CAF, cancer cell and PBMCs measured over a representative z-stack image showing the spatial cellular repartition in the co-culture.
**Figure 5e****.** Representative immunohistochemistry image of a-SMA
**Figure 6a.** Shows a fluorescence image of CAFs and PBMCs in a MIRO channel.
**Figure 6b.** Shows a time-lapse imaging of PBMC migrating in a MIRO channel colonized by CAFs. Scale bars: 100 µm.
**Figure 6c, left.** Shows a representative confocal fluorescent image showing a single PBMC (fire LUT) migrating (tracks, white) in the EDGE region.
**Figure 6c, right.** Shows a lateral view of the same z stack time-lapse imaging showing the PBMC migrating within the CAF layers. Scale bars: m. 20 µm, n. y-axis:10 µm; z-axis: 30 µm.
**Figure 6d.** Shows a fluorescence image of CAFs, PBMCs and fibronectin in a MIRO channel on day 10. Scale bars: 100 µm.
**Figure 6e.** Shows a fluorescence image of CAFs, PBMCs and fibronectin in a MIRO inlet on day 10. Scale bars: 100 µm.
**Figure 6f.** Shows a quantification of immune cell infiltration in the tumour islets.
**Figure 6g.** Shows a time projection of time-lapse imaging of PBMCs reaching MIRO inlet and interacting with the tumour spheroid. Scale bar: 100 µm.
**Figure 6h.** Shows a fluorescence image of the CAF, tumour spheroid and PBMC co-cultured in MIRO showing the area of measurement of the fluorescence intensity of PBMC quantified in (d).
**Figure 6i.** Shows the measurement of the relative intensity fluorescent signal over time of PBMCs exiting the channels in the presence or absence of the stroma. Scale bar: 200 µm.

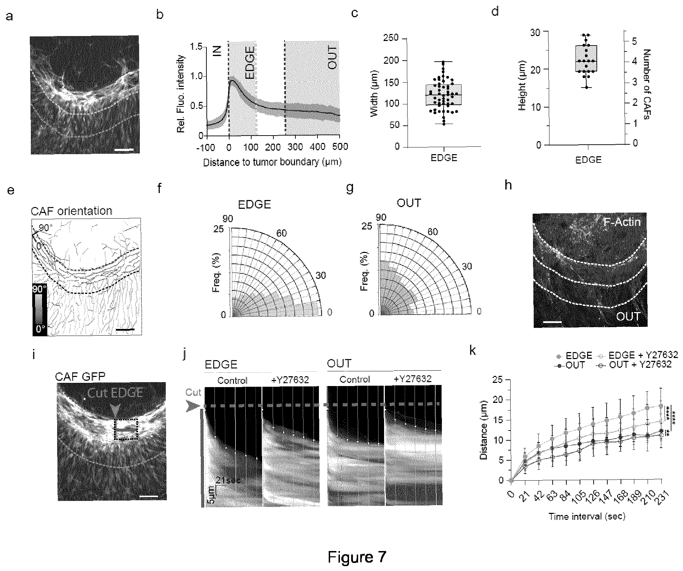

**Figure 7a****.** Shows a representative fluorescence image of CAFs repartition at the tumour/stroma boundary delineating the EDGE region, and the OUT-region boundary. Scale bar: 100 µm.
**Figure 7b****.** Shows relative CAFs fluorescent intensity profile averaged on z-projection of 41 images displaying the EDGE, IN and OUT regions delimitation relative to the tumour boundary.
**Figure 7c****.** Shows a quantification of the EDGE width based on z-projected fluorescent images of CAF-GFP signal.
**Figure 7d****.** Shows a quantification of the height of the EDGE region and calculation of the corresponding cell number based on z-stacked CAF GFP images.
**Figure 7e****.** Shows the orientation of CAFs at the EDGE
**Figure 7f****.** Shows the CAF orientation in comparison with the EDGE boundary of the cancer islet boundaries
**Figure 7g****.** Shows the CAF orientation in comparison with the OUT boundary of the cancer islet boundaries
**Figure 7h****.** Shows the signal distribution of the immunostaining effect of F-actin along the tumour/stroma boundary
**Figure 7i****.j.k.** Shows the disruptions of the TME in both the EDGE and OUT regions with a custom-made laser ablation system in the absence or presence of a myosin inhibitor (Y27632).

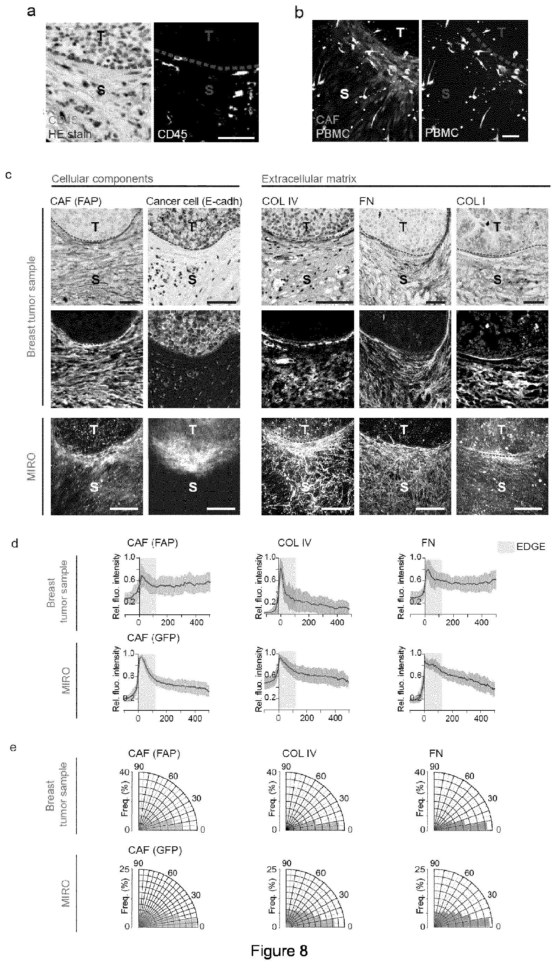

**Figure 8a****.** Shows an immune cell distribution in breast tumour samples (CD45 staining). Scale bars: 50 µm.
**Figure 8b****.** Shows an immune cell distribution in MIRO coculture (cell tracker labelled-PBMCs, fire-LUT). Scale bars: 50 µm.
**Figure 8c****.** Shows a immunohistochemistry and immunofluorescent staining targeting CAFs (IHC: FAP; IF: cytosolic GFP marker), tumour cells (E-cadherin (E-cadh)), ECM components: collagen IV (COL IV), fibronectin (FN) and collagen I (COL I). Scale bars: 100 µm.
**Figure 8d****.** Shows a relative fluorescent intensity profile of CAFs (GFP in MIRO, FAP in tumour samples) collagen IV (COL IV) and fibronectin (FN) in breast tumour samples.
**Figure 8e****.** Shows a distribution of the relative orientation of CAFs and ECM components (collagen IV and fibronectin) in breast tumour sample respective to the tumour/stroma boundary (set as 0 angle value), and in MIRO.

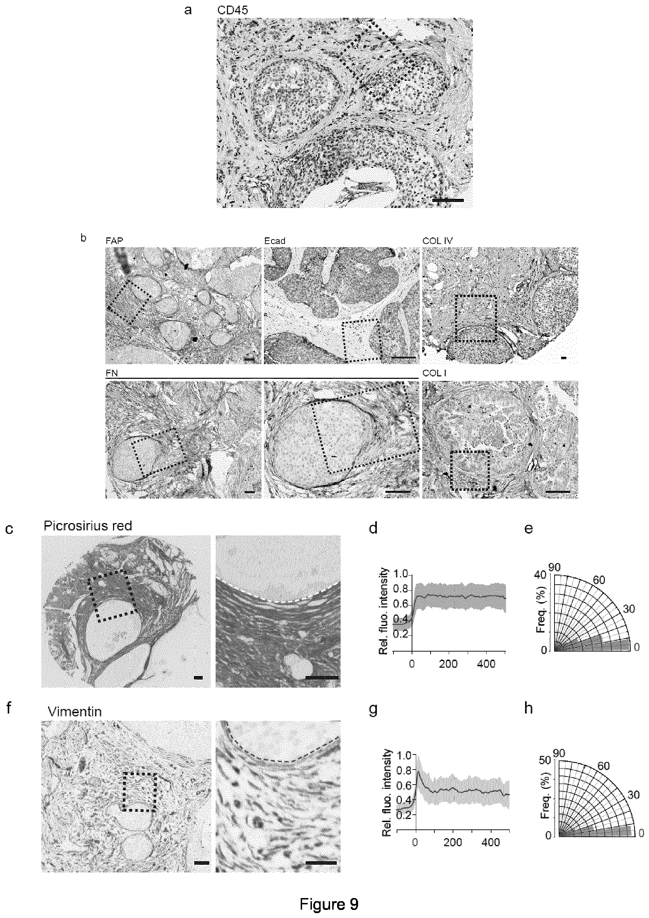

**Figure 9a****.** Shows an immunohistochemical detection results of CD45 immunofluorescent.
**Figure 9b****.** Shows an immunohistochemical detection results of CAF markers (fibroblast activation protein, FAP; vimentin, VIM), an epithelial marker (E-cadherin, E-cadh) and matrix proteins commonly found in solid tumours, such as collagens and fibronectin (collagens, Picrosirius red staining; collagen IV, COLIV; collagen I, COLI; fibronectin, FN).
**Figure 9c****.** Shows representative images of Picrosirius red (collagen) detection by IHC in ex vivo culture of patient-derived tumour samples.
**Figure 9d****.** Shows the quantification of the relative fluorescence intensity of the picrosirius red signal in the ex-vivo culture of derived patient-derived tumour samples.
**Figure 9e****.** Shows the quantification of the relative fluorescence relative orientation of the picrosirius red signal in the ex-vivo culture of derived patient-derived tumour samples.
**Figure 9f****.** Shows representative image of Vimentin detection by IHC in ex vivo culture of derived patient-derived tumour samples.
**Figure 9g****.** Shows the quantification of the relative fluorescence relative orientation of the vimentin signal in ex vivo culture of derived patient-derived tumour samples.
**Figure 9h****.** Shows the quantification of the relative fluorescence intensity of the vimentin signal in ex vivo culture of derived patient-derived tumour samples.

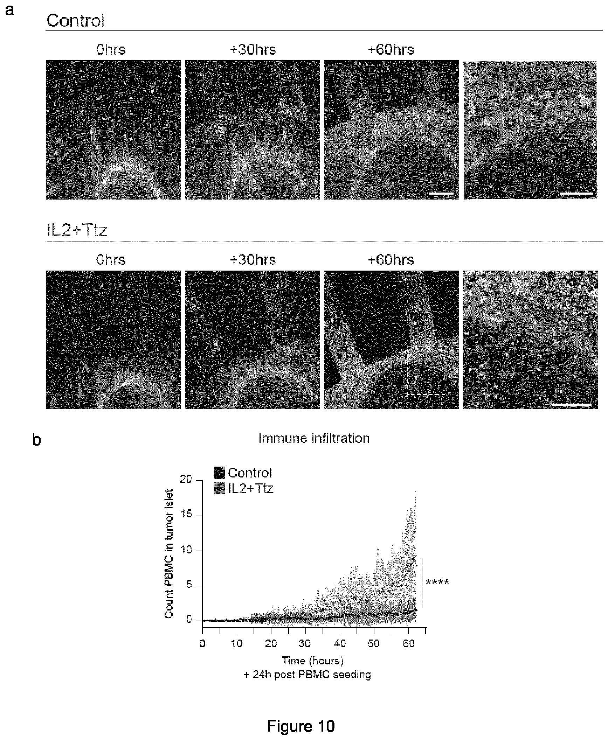

**Figure 10a****.** Shows a time lapse screenshot images showing PBMCs infiltrating into the tumour in the absence and in the presence of IL2+Ttz treatment. Scale bars: 200 µm; zooms: 100 µm.
**Figure 10b****.** Shows a quantification of immune cell infiltration in tumour islets 24 hours after PBMCs seeding in the absence (control, grey dots) or presence of IL2+Ttz treatment (pink dots)).

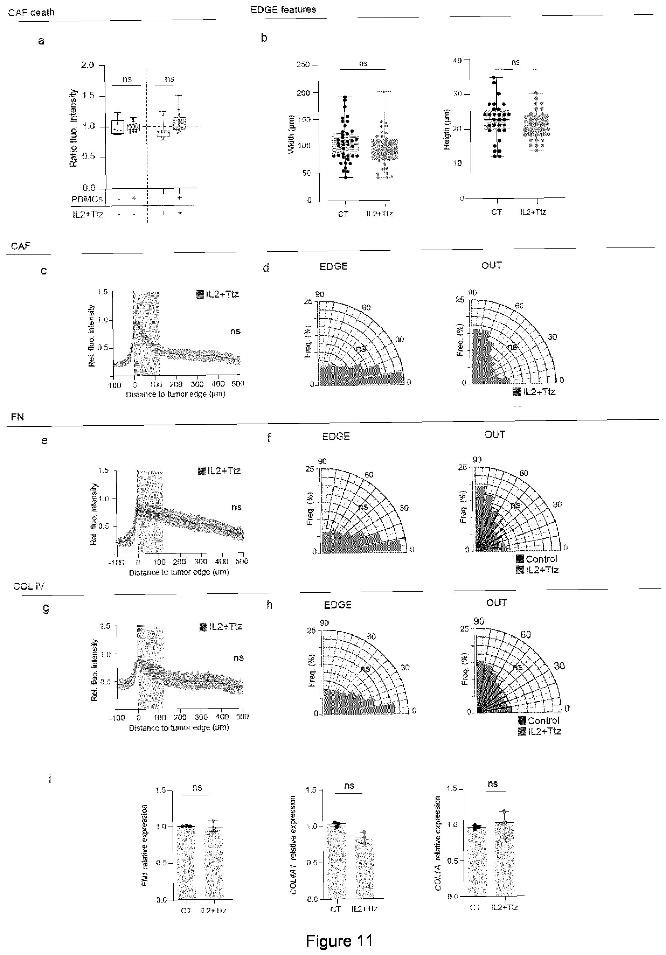

**Figure 11a****.** Shows a CAF cell death quantification.
**Figure 11b****.** Shows a Quantification of the width and height of the EDGE region of fixed samples based on CAFs GFP fluorescent signal in the presence or absence of IL2+Ttz treatment.
**Figure 11c****.e.g.** Shows fluorescence intensity profiles in MIRO of CAFs (c), FN (e) and COL IV (g.) at the tumour/stroma boundary (EDGE region, orange box) in the presence or absence of IL2+Ttz treatment
**Figure 11d****.f.h.** Shows the orientation of CAFs, FN and COL IV fibres respective to the tumour/stroma boundary in the EDGE and OUT region in presence of IL2+Ttz treatment and compared to control.
**Figure 11i****.** Shows a qRT-PCR quantification of FN (FN1), COL IV (COLIVA1) and COL I (COLIA1) expression in CAF monoculture upon IL2, Ttz, IL2+Ttz treatment or in absence of drugs (Control).

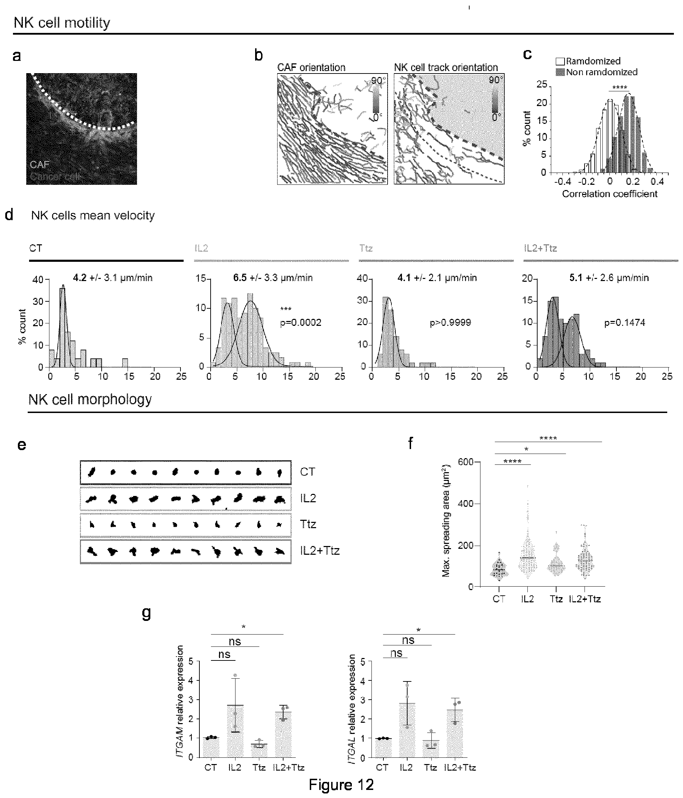

**Figure 12a****.** Shows a representative fluorescence image of CAF and cancer cells monitor over 90 min time-lapse acquisition in the tumour/stromal boundary region.
**Figure 12b****.** Shows an Orientation of CAFs and NK cell tracks relative to the tumour/stroma boundary.
**Figure 12c****.** Shows a correlation analysis of CAFs and NK cell tracks orientations in the EDGE region (non-randomized data, green bars) compared to randomized data correlation analysis (black bars and gaussian fit).
**Figure 12d****.** Shows a frequency distribution (in %) of NK cells mean velocity (v) from instant velocities in the tumour EDGE region upon IL2 treatment.
**Figure 12e****.** Shows a representative time-lapse binarized images of single NK cells migrating in the EDGE region in the presence or absence of treatment.
**Figure 12f****.** Shows a Maximum NK cells spreading area (m) of NK cells tracked in the tumour EDGE in MIRO.
**Figure 12g****.** Shows a qRT-PCR quantification of ITGAL, ITGAM expression level in NK cells co-cultured with CAFs in presence of IL2, Ttz, or IL2+Ttz and without treatment (CT).

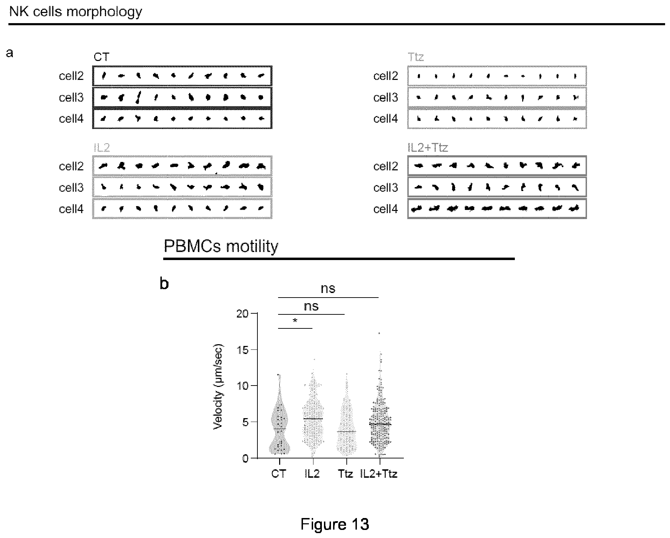

**Figure 13a****.** Shows representative sequences extracted from time-lapse binarized images of single NK cells migrating in the EDGE region in the presence or absence of treatment
**Figure 13b****.** Shows PBMCs cells mean velocity (v) in the tumour EDGE region in presence of IL2, Ttz or IL2+Ttz and without treatment.

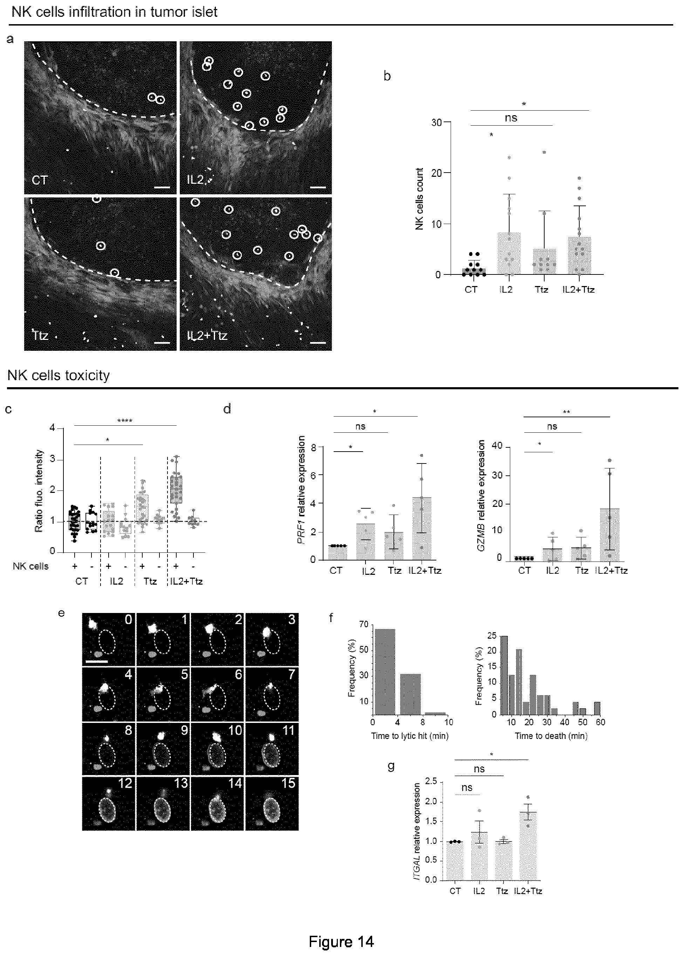

**Figure 14a****.** Show representative images of tumour/stroma boundary region in MIRO in the presence (IL2, Ttz, IL2+Ttz) or absence (CT) of treatment. Scale bars: 100 µm.
**Figure 14b****.** Shows NK cells counting in the tumour islet.
**Figure 14c****.** Shows a cancer cell death quantification. Quantification of cell death reporter FITC fluorescent signal in cancer cells in the absence (black boxes) or presence of IL2 (orange boxes), Ttz (blue boxes) or IL2+Ttz (pink boxes) drug treatments, and in the absence (white boxes) or presence (filled boxes) of NK cells.
**Figure 14d****.** Shows a qRT-PCR quantification of Perforin 1 (PRF1), and Granzyme B (GZMB) expression level in NK cells co-culture with tumour cells in the absence (CT) or presence of IL2, Ttz, or IL2+Ttz treatment and without treatment as indicated.
**Figure 14e****.** Shows a time-lapse imaging sequence of tumour cell death cancer cell nuclear contour (dashed white line) triggered by an NK cell (fire LUT) in MIRO. Scale bars: 5 µm.
**Figure 14f****.** Shows a frequency distribution of the duration of the lytic hits induced by NK cell and time to death quantification and measured upon IL2+Ttz treatment.
**Figure 14g****.** Shows an ITGAL expression measured by qRT-PCR in NK cells co-cultured with tumour cells in absence of treatment (CT) or upon IL2, Ttz, or IL2+Ttz treatment.

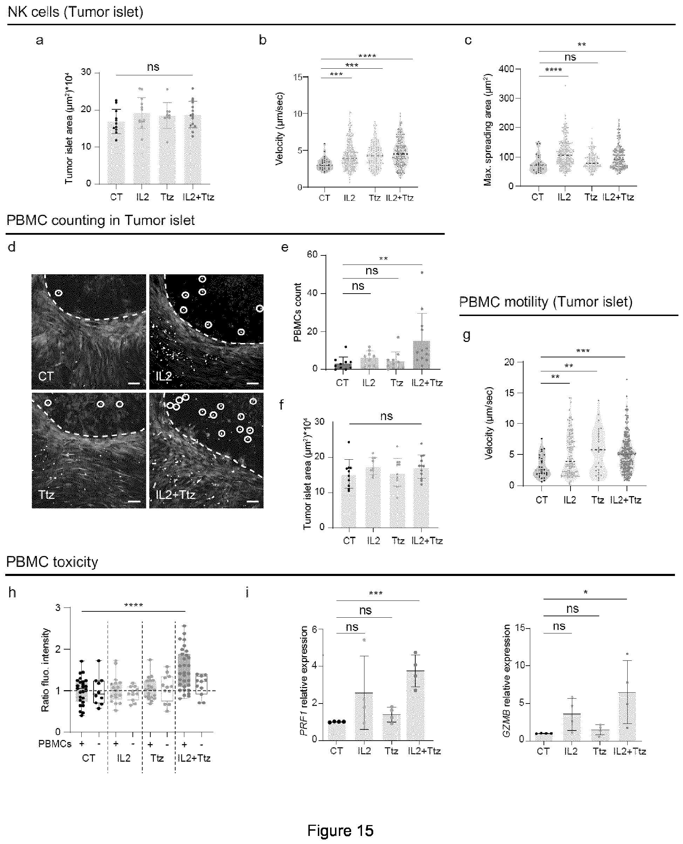

**Figure 15a****.** Shows a quantification of the tumour islet area in the absence (CT) or presence of IL2, Ttz, or IL2+Ttz treatment.
**Figure 15b****.** Shows NK cells mean velocity (v) in the tumour inlet in the absence (CT) or presence of IL2, Ttz, or IL2+Ttz treatment.
**Figure 15c****.** Shows a maximum spreading area (m) of NK cells tracked in the tumour inlet in MIRO in the absence (CT) or presence of IL2, Ttz, or IL2+Ttz treatment.
**Figure 15d****.** Shows a representative fluorescence image of the tumour/stroma region in MIRO in the absence (CT) or presence of IL2, Ttz, or IL2+Ttz treatment. Scale bars: 100 µm.
**Figure 15e****.** Shows a PBMCs counting in the tumour islet in the absence (CT) or presence of IL2, Ttz, or IL2+Ttz treatment.
**Figure 15f****.** Shows a relative tumour islet area in the presence or absence (CT) or presence of IL2, Ttz, or IL2+Ttz treatment.
**Figure 15g****.** Shows PBMCs cells mean velocity (v) in the tumour islet in the absence (CT) or presence of IL2, Ttz, or IL2+Ttz treatment.
**Figure 15h****.** Shows a cancer cell death quantification in the absence (CT) or presence of IL2, Ttz, or IL2+Ttz treatment.
**Figure 15i****.** Shows a qRT-PCR quantification of Perforin 1 (PRF1) and Granzyme B (GZMB) expression level in PBMCs co-cultured with tumour cell in the absence (CT) or presence of IL2, Ttz, or IL2+Ttz treatment.

### DESCRIPTION OF EMBODIMENTS

Now, preferred embodiments of the present application will be described in detail with reference to the annexed drawings. However, they are not intended to limit the scope of this application.

The present invention relates generally to the field of cell biology and biomedicine, more precisely to cell culture systems and cell culture methods which allow to effectively recapitulate the organisation and composition of the stroma, including fibroblasts and fibroblast-derived extracellular matrix cancer-associated fibroblast (CAFs) and cancer-associated fibroblast-derived extracellular matrix (CAF-derived ECM), found at the interface of solid tumours and around tissues and organs.

The cell culture system (1) of the present invention comprises the following zones as seen in Figure 1 to 5:
a body of the device (2) separating a first zone (3) and a second zone (4),
said body of the device (2) comprises at least one microchannel (5) fluidly communicating the first zone (3) with the second zone (4) and the at least one microchannel (5) comprises a first communicating end (6) in communication with the first zone (3) and a second communicating end (7) in communication with the second zone (4).

In one embodiment, the cell culture system (1) is characterized in that the shape (length (13), height (12) and width (11)) of the at least one microchannel (5) enables the flowing of a fluid without requiring an active pumping of said fluid.

In one embodiment, the cell culture system (1) is characterized in that the body of the device (2) surrounds the first zone (3).

In one embodiment, the cell culture system (1) is characterized in that the second zone (4) surrounds the body of the device (2).

In one embodiment, the cell culture system (1) is characterized in that the body of the device (2) defines or has the form of a circle.

In one embodiment, the cell culture system (1) is characterized in that the body of the device (2) has an inner radius (9) of up to 3 mm and an outer radius (10) of up to 5mm. In a preferred embodiment, the body of the device (2) has an inner radius (9) of 3 mm and an outer radius (10) of 5mm.

In one embodiment, the cell culture system (1) is characterized in that the first zone (3) has a shape suitable to be sealed into the glass/plastic bottom surface (14) by plasma treatment. In a preferred embodiment, the first zone (3), has a circular form.

In one embodiment, the cell culture system (1) is characterized in that the first zone (3) has a minimum radius of 3 mm, preferably of 6 mm.

In one embodiment, the cell culture system (1) is characterized in that the body of the device (2) comprises at least one microchannel (5) In a preferred embodiment, the cell culture system (1) comprises 50 microchannels (5).

In one embodiment, the cell culture system (1) is characterized in that at least one microchannel (5) has a width (11) between 1 and 300 µm, preferably of 200 µm.

In another embodiment, the cell culture system (1) is characterized in that at least one microchannel (5) has a width (11) selected from 5 or 200 µm.

In one embodiment, the cell culture system (1) is characterized in that at least one microchannel (5) has a minimum height (12) of 1 µm, preferably between 1 to 200 µm.

In another embodiment, the cell culture system (1) is characterized in that at least one microchannel (5) has a minimum height (12) selected from 10, 50 or 130 µm.

In one embodiment, the cell culture system (1) is characterized in that at least one microchannel (5) has a minimum length (13) of 0.5 mm, preferably between 1 and 3 mm.

In one embodiment, the cell culture system (1) is characterized in that the body of the device (2) is made of a biocompatible material. In one embodiment, the biocompatible material is a biocompatible polymer. In a preferred embodiment, the biocompatible polymer is selected from biocompatible synthetic polymers or biocompatible natural polymer.

In one embodiment, the biocompatible synthetic polymer is selected from, but not limited to: polydimethylsiloxane (PDMS), poly(methyl methacrylate) (PMMA), polycarbonate (PC), polystyrene (PS), polyvinyl chloride (PVC), polyimide (PI), the family of cyclic olefin polymers (i.e., cyclic olefin copolymer (COC), cyclic olefin polymer (COP), and cyclic block copolymer (CBC)) or thermoset polyester (TPE). In a preferred embodiment, the biocompatible synthetic polymer is polydimethylsiloxane (PDMS).

In one embodiment, the biocompatible natural polymer is chitosan.

The present application also refers to a method of producing the cell culture system (1) comprising the following steps:
- Producing the body of the device (2) by mixing polydimethylsiloxane PDMS with a curing agent in a 10:1 weight-to weight ratio;
- Pouring a mixture into a mould comprising at least one microchannel (5) to form a body of the device (2);
- Curing the body of the device (2) for a temperature between 55 and 85°C and a time between 1 and 12 hours;
- Peel the body of the device (2) from the mould;
- Cut the body of the device (2) to form the first zone (3) and the second zone (4);
- Activating the body of the device (2) and the glass/plastic bottom surface with plasma to enable the sealing of the body of the device (2) with a surface (14).

The obtained cell culture system (1) is placed in a well of a well plate (18) and sealed by means of plasma treatment. The cell culture system (1) is then made hydrophilic by means of corona treatment to ease the flow of liquid into the microchannels (5) of the cell culture system (1). The cell culture system (1) is then sterilised by exposure to ultraviolet light.

In one embodiment, the cell culture system (1) is characterized in that the body of the device (2) is made by mixing a silicone elastomer base PDMS and a silicone elastomer curing agent in a 10:1 weight to weight ratio.

In one embodiment, the cell culture system (1) is characterized in that the material of the body of the device (2) is suitable to be activated using oxygen plasma.

In one embodiment, the cell culture system (1) is characterized in that the cell culture system (1) is bound to a surface (14) of a cell culture plate or a well plate (18), preferably sealed to a surface (14).

In a preferred embodiment, the cell culture system (1) is sealed to a surface (14), preferably by using a surface plasma treatment.

In one embodiment, the cell culture system (1) is characterized in that the surface (14) is made of a biocompatible material. In a preferred embodiment, the biocompatible material is glass.

In one embodiment, the cell culture system (1) is characterized in that it is perimetrically delimited by a wall (17) of a cell culture plate or a well plate (18). In one embodiment, the cell culture system (1) is characterized in that the wall (17) is made of a biocompatible material. In one embodiment, the cell culture system (1) is characterized in that the wall (17) is made of biocompatible material such as plastic that is suitable for cell culture.

In one embodiment, the cell culture system (1) is characterized in that the surface (14) and the wall (17) are part of a cell culture plate or a well plate (18).

In one embodiment, the cell culture system (1) is characterized in that the cell culture system (1) is bound to the cell culture plate or well plate (18) preferably by sealing means, preferably by using a surface plasma treatment.

Due to the tuneable size of the cell culture system (1) of the present invention, it is fully compatible with multi-well plate, for example 12, 24 and 48 well plate, or multidevice per wells, up to 4 per 14mm diameter single petri dishes, enabling multiple drug screening for example.

In one embodiment, a cell culture plate with 12 wells is used, each of the wells comprises a cell culture system (1) of the present invention, as shown in Figure 6.

The present invention also relates to a method for cell culturing comprising the following steps, and as can be seen in Figure 7:
a) Providing a cell culture system (1) in accordance with any of the previous embodiments;
b) Seeding a first cell type in the inlet chamber (15) of the first zone (3);
c) Seeding a second cell type on top of the first cell type in the first zone (3).

In one embodiment, the method for cell culturing is characterized in that the first cell type are fibroblasts. In a preferred embodiment, the fibroblasts used are CAFs.

In one embodiment, the method for cell culturing is characterized in that the second cell type are cancer cells or epithelial cells. In one embodiment, the method for cell culturing is characterized in that the second cell type is provided in a multicellular structure (8), preferably in structures of at least 3 cells, such as spheroid, aggregates, cells organoids or tumoroids.

In one embodiment, the method for cell culturing is characterized in that step b) occurs at day 0.

In one embodiment, the method for cell culturing is characterized in that the time between step b) and step c) lasts for a time suitable for the first cell type to form a monolayer (19) that covers the surface of the first zone (3), a time up to 4 days. Preferably, step c) occurs at day 4.

In one embodiment, the method for cell culturing is characterized in that, it further comprises a step d) after step c), wherein step d) is:
d) application of a treatment in the outlet chamber (16) of the second zone (4) so that said treatment passes through the at least one microchannel (5) to the inner chamber (15) of the first zone (3).

In one embodiment, the method for cell culturing is characterized in that the treatment may be immunological cells and/or at least one drug.

In one embodiment, the method for cell culturing is characterized in that the time between step c) and step d) lasts for up to 2 days. Preferably, step d) occurs at day 6.

In one embodiment, the method for cell culturing is characterized in that the treatment is made with immune cells which can be seeded together with a drug treatment or treated before seeding in step d) in the outlet chamber (16) of the second zone (4) on day 6 and they infiltrate the at least one microchannel (5) reaching the inlet chamber (15).

In one embodiment, and up to 2 days after step d), occurs a spatial reorganization of the cells surrounding the multicellular structure (8), fluorescently labelled immune cells such as immune cell lines, peripheral blood mononuclear cells (PBMCs) (20), engineered immune cell such as CAR T cells or NK CAR cells can then seeded in the outlet chamber (16) together with drug treatment.

In one embodiment, imaging is performed using a confocal microscope between days 7 and 10 after immune cell seeding.

The results obtained are shown below to demonstrate that the present invention is not only useful for the generation of interfaces tumour/stroma but in general for the generation of interfaces tissue-organ/stroma, this is, tissue-organ margins, and, hence, also allows, for example, the study of immune infiltration in inflammatory processes within organs, for example, lung, Intestine, colon, bladder/stroma interface.

### Examples

### Example 1. Manufacture of a cell culture system (1) of the present invention

A mixture of polydimethylsiloxane (PDMS) and curing agents (Silicone Elastomer Kit) was mixed well in a 10:1 weight-to-weight ratio and poured on a resin stamp (the stamp comprised 50 microchannels (5) of 200µm width (11) and 50 µm height (12)), degassed in a vacuum desiccator, and solidified at 82°C for 2h. Then, the PDMS was peeled from the resin stamp, and 10 to 6 mm holes were punched (which will define the first zone (3) and second zone (4) of the cell culture system (1) of the present invention and the circular form of the body of the device (2) which defines a circle with an inner diameter of 6mm and an outer diameter of 10mm).

The PDMS was then cleaned from PDMS debris and activated using oxygen plasma (High Power Expanded Plasma Cleaner, Harrik Plasma; pressure 35 mg, 40 seconds).

The obtained body of the device (2) was then sealed on a 12-well plate by means of plasma treatment, with one barrier occupying each well. Next, the bodies of the device were made hydrophilic with corona treatment to ease the flow of liquid into the channels of the device.

The cell culture systems (1) of the present invention obtained in this example were then sterilised with exposure to ultraviolet light for 15 min.

### Example 2. Generation and analysis of an interface tumour-stroma on the basis of a method and cell culture system (1) of the present invention

Cell culture systems (1) as obtained in example 1 were used.

Figure 7 shows an overview of the cell culture method carried out in the present example.

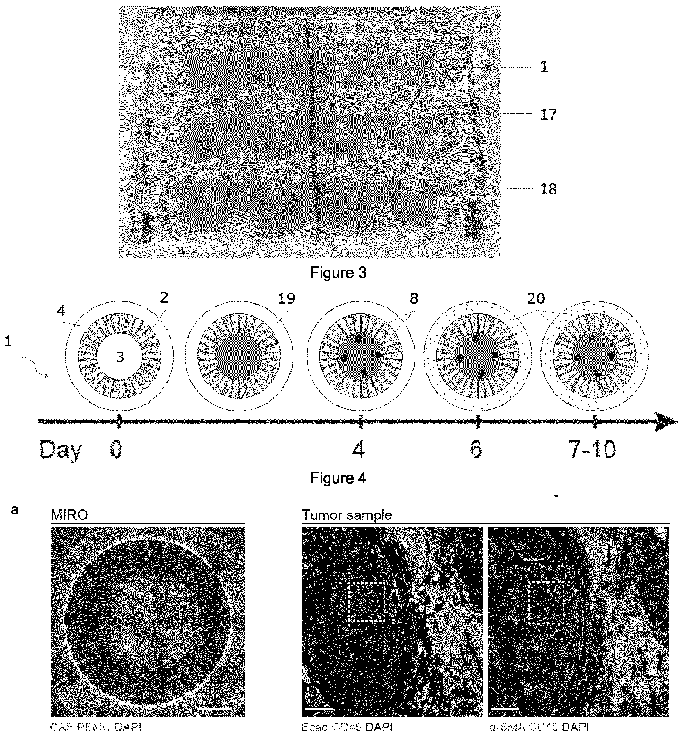

First, the first zone (3) and the second zone (4) of the cell culture system (1) of the present invention were equilibrated with a total of 1 ml media. Then fluorescently labelled and immortalized cancer associated fibroblasts (CAFs) from human breast tumours were cultured in the first zone (3) and left to grow until fully covering the surface of the said first zone (3) and the microchannels (5). m-cherry-labelled HER2+ breast cancer cells (H1954) spheroids were then seeded on top of the CAF layer at the proximity of the channel entries (day 4). The concomitant proliferation of CAFs and cancer cells generates a three-dimensional interface (EDGE) that separated the tumour core (IN) from the stromal region (OUT) and which correctly reproduced the interface tumour-stroma (this is, the cancer margin). After two days of co-culture, treatment was applied through the second zone (4), in this case peripheral blood mononuclear cells (PBMC) (20), purified from the blood of healthy donors and previously labelled. PBMCs (20) entered the channels and, 24 hours after seeding, they reach the first zone (3) of the cell culture system (1) of the present invention (Fig. 4).

The device is sealed onto a glass-bottom dish through plasma treatment. Then, fluorescently labelled and immortalized CAFs from human breast tumours are cultured until fully populating the surface of the said first zone (3) and the channels connecting to the second zone (4). mCherry-labelled HER2+ breast cancer cells (H1954) spheroids are then seeded on top of the CAFs layer at the proximity of the channel entry (Fig. 5a,b). During the next two days, CAFs and cancer cells proliferate and self-organize to generate a 3D interface (EDGE) that segregates cancer islets (IN) from the stromal compartment (EDGE + OUT) (Fig. 5c,d). Two days after cancer cells seeding on top of CAFs, primary immune cells (peripheral blood mononuclear cells, PBMCs) purified from the blood of healthy donors, or natural killer cells (NK cells) derived from PBMCs are labelled with a fluorescent cell tracker and inoculated in the second zone (4) (Fig. 5a). Approximately 2 days after seeding immune cells stand mostly excluded from the epithelial cancer cell islets and accumulate in the stroma in this experimental setting (Fig. 5b-d).

It was observed that the spatial organization of CAFs at cancer/stroma interface closely resembles similar regions found in solid tumours (Fig. 5a,b right panels; Fig. 5e). This spatial organization resembles the immune excluded tumour phenotype, which is known from the state-of-the-art to be often associated with poor prognosis in multiple cancer types¹.

In vivo observations shows that fibrotic barriers mainly formed by CAF and the ECM surrounding epithelial tumour nests strongly affect immune cell trafficking in tumours¹⁴. In the model obtained, time-lapse imaging of PBMCs migrating in MIRO channels and the EDGE region colonised by CAFs reveals tight interactions with the stromal layers (Fig. 6a-c). Additionally, immunostainings of fibronectin, a CAF-derived ECM protein found in many solid tumours, show an abundant deposition in the MIRO inlet chamber (15) and the channels colonised by CAFs (Fig. 6d,e).

To address the functional role of CAFs and CAF-derived ECM in immune exclusion, the intraepithelial immune infiltration in cancer islets in the presence or absence of CAFs were compared. In this setting, immune infiltration is monitored by fluorescence live confocal microscopy for 60 hours (1 frame every 15 minutes), starting 24 hours after the addition of PBMCs (t0). The obtained data revealed that immune cells infiltrate the tumour islet unopposed in the absence of stroma, leading to a 4-fold higher immune infiltration compared with the experiments in the presence of stroma (Fig. 6f,g).

Then, it was assessed the presence of PBMC in the vicinity of the tumour in both conditions by measuring the fluorescent signal of PBMC at the exit of the channels. The results obtained show that both in the presence and absence of stroma PBMC fluorescent signal is detectable around 30 hours after PBMC seeding into the device (Fig. 6h,i). These findings show that in the MIRO platform CAFs and their secreted ECM contribute to immune exclusion, as already reported in previous studies in patients¹⁴, and that immune cells actively migrate within the stroma in the EDGE region.

Then, a characterization of the geometry, composition, and mechanics of the EDGE was made. Intensity profile analyses of GFP-tagged CAFs (Fig. 7a,b) indicate that this region had a width (11) of 120 µm ± 33 µm and height (12) of 24 µm ± 5 µm (mean ± SD), equivalent to the thickness of 4 ± 1 cells (Fig. 7c,d). To measure the orientation of CAFs at the EDGE, the projections of z-stack images of GFP-tagged CAFs were analysed (Fig. 7e). These results showed that CAF orientation tightly follows the edge of the cancer islet boundaries (Fig. 7f), resembling the stromal organization surrounding cancer islets from diverse tumours origins^{14, 31}. In contrast, CAFs located in more distal regions (OUT, 120 µm from the EDGE) preferentially orient perpendicularly to the EDGE (Fig. 7g). Accordingly, immunostaining of F-actin shows a higher intensity signal along the tumour/stroma boundary (Fig. 7h), supporting these findings of preferential orientation and compactness of CAFs at the proximity of the tumour islet. This observation suggests that the EDGE could bear higher tension than the OUT region as suggested in the state of the art^{32, 33}.

To test this hypothesis, local disruptions of the TME in both the EDGE and OUT regions with a custom-made laser ablation system (Fig 7i-k) was performed. These data indicate that EDGE-CAFs recoil faster and to a greater extent than OUT-CAFs distant from the cancer islet. In addition, the significant reduction of the recoiled distances in presence of a myosin inhibitor (Y27632) in the EDGE suggests that CAFs actomyosin contractility might play a major role in maintaining the mechanical and structural properties of the stroma around the tumour islet in this model.

Then, it was assessed the extent to which MIRO mimics the composition, organization and morphology of the cellular components and ECM observed in patients at the tumour/stroma interface. Firstly, a comparison of the distribution of immune cells in fixed samples of both patient tumour samples and MIRO was made, which shows that in both cases immune cells are largely excluded from the epithelial cancer cell islets and align with the cancer/stroma interface (Fig. 8a,b). Then, it was carried out a comparative histopathological analysis of breast tumour samples and MIRO. For this, it has been performed a immunohistochemical detection of CAF markers (fibroblast activation protein, FAP; vimentin, VIM), an epithelial marker (E-cadherin, E-cadh) and matrix proteins commonly found in solid tumours, such as collagens and fibronectin (collagens, Picrosirius red staining; collagen IV, COLIV; collagen I, COLI; fibronectin, FN) (Fig. 8c; Fig. 9b,c).

The obtained data shows that MIRO and patient tumour samples display comparable CAF/cancer cell organization as well as a very similar pattern of COLIV, FN and COLI deposition (Fig. 8c). Quantitative analysis of the relative fluorescent intensity plots from cumulative z-stack confocal imaging indicates that patient tumours and MIRO co-cultures also share similar distribution of CAF (FAP and VIM) collagens, COLIV, FN (Fig. 8d; Fig. 9c,d,f,g). The intensity profiles at the EDGE regions in the MIRO assay and patient tumours show similar width (11) and a higher density of CAFs and ECM compared to distal regions (Fig. 8c,d; Fig. 9d,g). Image analysis of fluorescence images using Curvalign and custom-made Matlab script shows that CAFs and ECM fibres align parallel to the cancer islet edges similarly both in MIRO assay and patient tumours EDGE regions (Fig. 8c,e; Fig. 9e,h).

Collectively, these data indicate that the TME in MIRO replicates the aberrant production and architecture of ECM observed in the vicinity of the cancer islets in patients as suggested by the state of the art³⁴.

### Example 3. Study of immune exclusion and infiltration in tumours by means of the cell culture system (1) and method of the present invention

Cell culture systems (1) as obtained in example 1 were used. In addition, the tumour/stroma interface was generated by means of the method explained in example 2.

In the present example, it was analysed the applicability of MIRO assay for drug testing using Trastuzumab (Ttz), a monoclonal antibody (mAb) anti-HER2. Has already known from the state of the art, and in addition to direct cytotoxicity of Ttz against HER2+ breast cancer cells³⁵, mAbs anti-HER2 have proven to robustly enhance anti-cancer immunity through ADCC^{36, 37}. Despite Ttz proven efficiency, recent state of the art studies reported that HER2+ breast cancer patients unresponsive to anti-HER2 mAbs developed an immunosuppressive phenotype suggesting a resistance to mAbs-induced ADCC³⁸.

To assess immune response mediated by Ttz treatment, MIRO with Trastuzumab in combination with interleukin-2 (IL2) (hereafter referred to IL2+Ttz) were inoculated, which is known from the state of the art to be a powerful immunomodulator used to treat patients with advanced cancers³⁹ (Fig. 10a).

As it is known from the state of the art, HCC1954 breast cancer cells implanted in MIRO are intrinsically resistant to anti-HER2 mAbs⁴⁰, that allows to assess the immunomodulatory effect of Trastuzumab using a low concentration of the drug. Both in treated and non-treated MIRO co-cultures, immune cells reach the tumour EDGE approximately 30 hours after seeding (Fig. 10a,b). With time, PBMCs remain mostly excluded from the cancer islets in the control condition whereas IL2+Ttz treatment significantly increases (7-fold) intraepithelial immune infiltration. This result suggests that the stromal barrier can be breached by immune cells upon specific cell targeting and effector cell activation (Fig. 10a,b).

Due to the impact of the stroma on immune cell trafficking, it is important to evaluate whether IL2+Ttz treatment could affect the architectural and molecular characteristics of the tumour stroma and therefore, the permeability of the stromal barrier. Firstly, it is quantified CAF apoptosis upon IL2+Ttz treatments by performing cell death assays in MIRO using a fluorescent red marker (Propidium iodide) in the presence or absence of PBMCs with and without IL2+Ttz treatment (Fig. 11a). The obtained results show no significant differences in CAF death rate upon drug treatment in presence or absence of treatment.

Along with CAFs viability results, the EDGE width (11) and height (12) also remained unaffected by IL2+Ttz treatment (Fig. 11b). It is then hypothesised that treatment could modulate CAF and ECM organisation, impacting CAF contact guidance, ECM assembly and immune cell trafficking, as suggested by the state of the art¹¹.

Quantitative analysis of FN and COLIV immunodetection after four days of treatment indicates no significant changes in the distribution and orientation upon Ttz+IL2 treatment compared to control conditions (Fig. 11c-h). Consistently, the expression levels of FN, COLI, and COLIV reveal no significant changes in cultured CAFs upon IL2+Ttz treatment (Fig. 11i). These data suggest that Trastuzumab and IL2 treatment does not impact the stroma composition and organization to explain immune infiltration in tumour islets in the invention model.

Then it is necessary to investigate the capacity of IL2, Ttz or IL2+Ttz treatments to modulate immune cell trafficking through the stromal barrier. As already known from the state of the art, an ADCC response is essentially mediated by NK cells⁴¹. Therefore, MIRO is inoculated with NK cells purified from PBMCs. Time-lapse imaging of single focal planes in MIRO (~1 frame/minute, for 90 minutes) enables tracking of a large majority of NK cells in the vicinity of the epithelial cancer cell islet (Fig. 12a). This approach makes it possible to monitor the orientation, velocity, and morphology of individual NK cell trafficking into the tumour stroma.

As several state-of-the-art reports highlighted, the scaffolding role of the stroma in guiding immune cells^{42, 43}, it is important to firstly analyse the correlation between the NK cell migratory paths and CAF alignment in our model. The time projection of NK cell trajectories was then overlayed on the CAF orientation map (Fig. 12b). For each track position, the closest CAF position was retrieved and the relative angle of both vectors at each position was saved as pairs. The correlation analysis performed on those pairs revealed a preferential migration of NK cells following the major elongation axis of CAF (Fig. 12b,c).

Similarly, previous state of the art studies showed that collagen alignment guides T cells migration around the tumour islets^{13, 14}. Then, it is assessed the effect of those treatments on the velocity of NK cells in the EDGE region. Compared to untreated cells, it is observed a significant increase by 55% of the NK cell average speed upon IL2 treatment, and by 21% upon IL2+ Ttz treatment but not in presence of Ttz treatment alone (Fig. 12f). Those data suggest that IL2 treatment is mainly responsible for the modulation of the migratory behaviour of NK cells.

IL2 treatment has been described in the state of the art to enhance integrin and F-actin expression, and lytic cellular behaviour^{44, 45}. Then, it is assessed the effect of drug treatment on the morphology of migrating NK cells.

To gain more insights into NK cell shape during migration, a time-lapse imaging was performed using z-stacks in the tumour/stroma interface region (1.5 min/frame, for 30 minutes). The z-projection of this time-lapse captures remarkable NK cell deformations while moving between tumour cells and CAFs. These results allow to quantify the maximum spreading area of each NK cell tracked in the different drug conditions masking each cell based on their fluorescent signal (cytosolic cell tracker) (Fig. 12e-g; Fig. 13a). The obtained results show that IL2 and IL2+Ttz treatments significantly increase NK cell area by 85% and 59% respectively compared to the control condition but not significantly in presence of Ttz alone (36%). In addition, it has been analysed the expression levels of integrins (ITGAM and ITGAL) whose implication in the shape and cellular migration of NK cells has been previously described in the state of the art^{46, 47}.

qPCR analysis of NK cells co-cultured with CAFs shows a significant increase of these migration markers upon IL2 and IL2+Ttz treatments supporting IL2 implication in NK migration towards tumours (Fig. 12g). Interestingly, a similar effect of IL2 and II2+Ttz treatment was observed, although non statistically significant on the average speed of unsorted PBMCs despite the heterogeneity of the immune cell populations in those samples (Fig. 13b). Overall, it is shown that MIRO enables to assess the effect of treatment on single cell behaviour in the tumour surrounding.

It has been found that IL-2 gives the immune cells a more migratory phenotype increasing their overall speed, integrin expression level and spreading capacity. Those results align with previous studies performed in vitro showing that IL2-activated NK cells have enhanced motility behaviour and dynamical morphology and cytotoxic capacity compared to resting NK cells^{44, 48}.

Then, MIRO is used to quantify the cytotoxicity of NK cells against cancer cells upon treatment. NK cell counting reveals that treatment with IL2 or IL2+Ttz increases infiltrated NK cells in tumour islets by -6-fold compared to the control (Fig. 14a,b; Fig. 15a). To assess whether higher immune infiltration in the cancer islet correlates with a higher rate of cancer cell death, the cytotoxic activity of NK cells in the different treatment conditions is quantified (Fig. 14c). To this end, it is used a fluorescent reporter of cell death.

It is observed an increase in cancer cell death upon Ttz monotherapy in the presence of NK cells which suggests the presence of a cytotoxic pool of NK cells in absence of IL2 treatment. Remarkably, the addition of IL2 to Ttz treatment increases drastically tumour cell death in presence of NK cells compared to IL2 or Ttz single therapy (Fig. 14c). Altogether, the obtained data suggest that IL2 boosts immune cell activation and NK cell migration through the CAF/ECM barrier. The resulting increase of intraepithelial immune cells in turn favours Ttz-induced ADCC and cancer cell eradication. Consistently, the analysis of ADCC markers by RT-qPCR in NK cells co-cultured with cancer cells indicates that the expression levels of Perforin and Granzyme B are significantly increased upon IL2 or IL2+Ttz treatment (Fig. 14d). These data suggest a higher cytolytic activity, cytokine secretion and degranulation of infiltrating immune cells upon IL2 administration.

As known from the state of the art, ADCC is a local mechanism that implies transient cell-cell contacts leading to targeted cellular membrane perforation⁴¹. To observe these transient events, a time-lapse imaging upon the addition of a fluorescent cell death reagent was performed (Fig. 14e). It is observed a significant number of ADCC events mediated by NK cells with short lytic contact upon IL2+Ttz treatment.

Remarkably, 67% of cancer cell contacts are under 8 minutes long leading to 72,5% of total tumour cell death in the first 20 minutes of time-lapse monitoring (Fig. 14e-g). These values are consistent with previous state of the art studies assessing NK cell driven ADCC response in 3D matrices⁴⁸.

During ADCC events, the immunological synapse enables NK cell degranulation upon ligand recognition and requires firm adhesion to trigger sufficient activating signaling. As integrin LFA1 (also known as ITGAL) is involved in the adhesion of lymphocytes and targeted cells during immunological synapses we assess its expression level in NK cells after 2 days of co-culture with HCC1954 and upon IL2, Ttz and IL2+Ttz treatments (Fig. 14f).

The obtained results show that IL2+Ttz treatment significantly upregulates ITGAL expression in NK cells (Fig. 14g). Next, the velocity and spreading area of NK cells in cancer islets is evaluated (Fig. 15c-d). In addition, the tracking of NK cells in the IN region indicates that the average speed upon IL2, Ttz and IL2+Ttz treatment was significantly increased compared to control suggesting a global increase in the scanning behaviour of NK cells in the tumour islet (Fig. 15c). IL2 and IL2+Ttz treatments also significantly increased the NK cell maximum spreading area respectively by 44% and 32% in the tumour islets compared to the untreated cells, and by 13% but not statistically significant in presence of Trastuzumab alone (Fig. 15d).

Performing similar studies using unsorted PBMCs shows similar effects of IL2 and IL2+Ttz treatments on immune infiltration, toxicity and motility compared to untreated samples (Fig. 15e-h).

Collectively, these data indicate that IL2 stimulates immune cell trafficking in tumours and restores Ttz-induced ADCC in immunosuppressive tumours.

Therefore, the results obtained in this example, showed that the cell culture system (1) and cell culture method of the present invention allowed for effectively recapitulate and reproduce the tumour/stroma interface (tumour margin), effectively recreating the stromal barrier, and providing for an effective instrument for the study of, for example, the influence and mechanisms of action of immune cells and treatments in tumours and their effectiveness.

### REFERENCES

1. Galon, J., et al. Approaches to treat immune hot, altered and cold tumours with combination immunotherapies. Nat. Rev. Drug Discov. 2018 183 18, 197-218 (2019).
2. Denkert, C. et al. Tumour-infiltrating lymphocytes and prognosis in different subtypes of breast cancer: a pooled analysis of 3771 patients treated with neoadjuvant therapy. Lancet Oncol. 19, 40-50 (2018).
3. Lundgren, S. et al. The Prognostic Impact of NK/NKT Cell Density in Periampullary Adenocarcinoma Differs by Morphological Type and Adjuvant Treatment. PLoS One 11, e0156497 (2016).
4. Subbiah, I.M., et al. Advances and future directions in the targeting of HER2-positive breast cancer: Implications for the future. Curr. Treat. Options Oncol. 15, 41-54 (2014).
5. Giraldo, N.A. et al. The clinical role of the TME in solid cancer. Br. J. Cancer 2018 1201 120, 45-53 (2018).
6. Vizoso, M. et al. Aberrant DNA methylation in non-small cell lung cancer-associated fibroblasts. Carcinogenesis (2015).
7. Labernadie, A. et al. A mechanically active heterotypic E-cadherin/N-cadherin adhesion enables fibroblasts to drive cancer cell invasion. Nat. Cell Biol. 19, (2017).
8. Takahashi, H. et al. Cancer-associated fibroblasts promote an immunosuppressive microenvironment through the induction and accumulation of protumoral macrophages. Oncotarget 8, 8633 (2017).
9. Linares, J., et al. Determinants and Functions of CAFs Secretome During Cancer Progression and Therapy. Frontiers in Cell and Developmental Biology 8, 621070 (2021).
10. Cox, T.R.. The matrix in cancer. Nat. Rev. Cancer 2021 214 21, 217-238 (2021).
11. Park, D. et al. Extracellular matrix anisotropy is determined by TFAP2C-dependent regulation of cell collisions. Nat. Mater. (2019).
12. Ray, A., et al. Aligned forces: Origins and mechanisms of cancer dissemination guided by extracellular matrix architecture. Curr. Opin. Cell Biol. 72, 63-71 (2021).
13. Sun, X., et al. Tumour DDR1 promotes collagen fibre alignment to instigate immune exclusion. Nat. 2021 5997886 599, 673-678 (2021).
14. Salmon, H., et al. Matrix architecture defines the preferential localization and migration of T cells into the stroma of human lung tumors. J. Clin. Invest. 122, 899-910 (2012).
15. Infanger, D.W., et al. Engineered Culture Models for Studies of Tumor-Microenvironment Interactions. Annu. Rev. Biomed. Eng. 15, 29-53 (2013).
16. Sunyer, R., et al. Collective cell durotaxis emerges from long-range intercellular force transmission. Science (80-.). 353, (2016).
17. Conti, S. et al. CAFs and cancer cells co-migration in 3D spheroid invasion assay. in Methods in Molecular Biology 2179, 243-256 (Humana Press Inc., 2020).
18. Yamada, K.M., et al, Cell-3D matrix interactions: recent advances and opportunities, Trends in Cell Biology, Volume 32, Issue 10 (2022)
19. Rodrigues, J., et al. 3D In Vitro Model (R)evolution: Unveiling Tumor-Stroma Interactions. Trends in Cancer 7, 249-264 (2021).
20. Sontheimer-Phelps A., et al, Modelling cancer in microfluidic human organs-on-chips. Nat Rev Cancer (2019)
21. Jeon J.S., et al. Human 3D vascularized organotypic microfluidic assays to study breast cancer cell extravasation. Proc Natl Acad. Sci USA (2015)
22. Moya M.L., et al. In vitro perfused human capillary networks. Tissue Eng Part C Methods, 19(9):730-7 (2013)
23. Choi, Y., et al. A microengineered pathophysiological model of early-stage breast cancer. Lab Chip. 15(16):3350-3357 (2015))
24. Parlato, S. et al. 3D Microfluidic model for evaluating immunotherapy efficacy by tracking dendritic cell behaviour toward tumor cells. Sci. Rep. 7, 1093 (2017).
25. Ayuso J.M., et al. Microfluidic tumor-on-a-chip model to evaluate the role of tumor environmental stress on NK cell exhaustion. Sci Adv. (2021)
26. Nguyen M., et al. Dissecting Effects of Anti-cancer Drugs and Cancer-Associated Fibroblasts by On-Chip Reconstitution of Immunocompetent Tumor Microenvironments. Cell Rep. (2018);
27. Lee S.W.L., et al. Characterizing the Role of Monocytes in T Cell Cancer Immunotherapy Using a 3D Microfluidic Model. Front Immunol. (2018))
28. Chauveau A., et al. Visualization of T Cell Migration in the Spleen Reveals a Network of Perivascular Pathways that Guide Entry into T Zones. Immunity. (2020)
29. Headley M.B., et al. Visualization of immediate immune responses to pioneer metastatic cells in the lung. Nature. (2016);
30. Crainiciuc G., et al. Behavioural immune landscapes of inflammation. Nature. (2022)
31. Brett E.A., et al. Tumor-associated collagen signatures: pushing tumor boundaries. Cancer Metab. (2020)
32. Kollmannsberger, P., et al. Tensile forces drive a reversible fibroblast-to-myofibroblast transition during tissue growth in engineered clefts. V. CELLBIOLOGY (2018).
33. Ferruzzi, J. et al. Compressive Remodeling Alters Fluid Transport Properties of Collagen Networks - Implications for Tumor Growth. Sci. Reports 2019 91 9, 1-16 (2019).
34. Gomes, R.N., et al. The bright side of fibroblasts: molecular signature and regenerative cues in major organs. npj Regen. Med. 2021 61 6, 1-12 (2021).
35. Swain, S. M. et al. Pertuzumab, trastuzumab, and docetaxel in HER2-positive metastatic breast cancer. N. Engl. J. Med. 372, 724-734 (2014).
36. Ahmed, S., et al. HER2-directed therapy: current treatment options for HER2-positive breast cancer. Breast Cancer 22, 101-116 (2015).
37. Hudis, C.A. Trastuzumab - Mechanism of action and use in clinical practice. New England Journal of Medicine 357, 39-51 (2007).
38. Force, J. et al. Early Stage HER2-Positive Breast Cancers Not Achieving a pCR From Neoadjuvant Trastuzumab- or Pertuzumab-Based Regimens Have an Immunosuppressive Phenotype. Clin. Breast Cancer 18, 410-417 (2018).
39. Amaria, R., et al. Update on use of aldesleukin for treatment of high-risk metastatic melanoma. ImmunoTargets Ther. 4, 79 (2015).
40. Von Heyde, S.D., et al. mRNA Profiling Reveals Determinants of Trastuzumab Efficiency in HER2-Positive Breast Cancer. PLoS One 10, e0117818 (2015).
41. Nigro, C. Lo et al. NK-mediated antibody-dependent cell-mediated cytotoxicity in solid tumors: biological evidence and clinical perspectives. Ann. Transl. Med. 7, 105-105 (2019).
42. Wolf, K., et al. Amoeboid shape change and contact guidance: T-lymphocyte crawling through fibrillar collagen is independent of matrix remodeling by MMPs and other proteases. Immunobiology, Blood, 102 (9): 3262-3269 (2003);
43. Bhattacharjee, O., et al. Unraveling the ECM-Immune Cell Crosstalk in Skin Diseases. Front Cell Dev Biol. (2019)
44. Olofsson, P.E. et al. Distinct migration and contact dynamics of resting and IL-2-activated human natural killer cells. Front. Immunol. 5, 80 (2014).
45. Orange, J.S. et al. IL-2 induces a WAVE2-dependent pathway for actin reorganization that enables WASp-independent human NK cell function. J. Clin. Invest. 121, 1535-1548 (2011).
46. Hornung, A. et al. A Bistable Mechanism Mediated by Integrins Controls Mechanotaxis of Leukocytes. Biophys. J. 118, 565 (2020).
47. Gismondi, A., et al. Migration of NK cells. Lymph. Traffick. Heal. Dis. 95-112 (2006).
48. Olofsson, P.E. et al. A collagen-based microwell migration assay to study NK-target cell interactions. Sci. Reports 2019 91 9, 1-10 (2019).

## Claims

1. A cell culture system (1) comprising:
- a body of the device (2) separating a first zone (3) and a second zone (4),
said body of the device (2) comprises at least one microchannel (5) fluidly communicating the first zone (3) with the second zone (4) and the at least one microchannel (5) comprises a first communicating end (6) in communication with the first zone (3) and a second communicating end (7) in communication with the second zone (4).

2. Cell culture system (1) according to the previous claim, wherein the body of the device (2) surrounds the first zone (3), and the second zone (4) surrounds the body of the device (2).

3. Cell culture system (1) according to any of the previous claims, **characterized in that** the body of the device (2) is made of a biocompatible material such as synthetic polymer, a biocompatible natural polymer, glass or metal.

4. Method of producing the cell culture system (1) described in any of the previous claims **characterized by** comprising the following steps:
- Producing the body of the device (2) by mixing polydimethylsiloxane PDMS with a curing agent in a 10:1 weight-to weight ratio;
- Pouring a mixture into a mould comprising at least one microchannel (5) to form a body of the device (2);
- Curing the body of the device (2) for a temperature between 55 and 85°C and a time between 1 and 12 hours;
- Peel the body of the device (2) from the mould;
- Cut the body of the device (2) to form the first zone (3) and the second zone (4);
- Activating the body of the device (2) and the glass/plastic bottom surface (14) with plasma to enable the sealing of the body of the device (2) with a surface (14).

5. A cell culture plate or a well plate (18) comprising at least one cell culture system (1) as disclosed in any of the claims 1 to 3.

6. Method for cell culturing using the cell culture system (1) described in any one of the previous claims **characterized by** comprising the following steps:
a) Providing a cell culture system (1) in accordance with any one of claims 1 to 3.
b) Seeding a first cell type in the inlet chamber (15) of the first zone (3);
c) Seeding a second cell type on top of the first cell type in the first zone (3).

7. Method according to the previous claim, wherein the first cell type is fibroblast.

8. Method according to claim 6, wherein the second cell type are cancer cells or epithelial cells.

9. Method according to any of the claims 6 to 8, wherein the method further comprises a step d) after step c), wherein step d) is the application of a treatment in the outlet chamber (16) of the second zone (4).

10. Method according to the previous claim, wherein the treatment is made with immunological cells and/or at least one drug.

11. Method according to any of the claims 6 to 10, wherein the time between step b) and step c) is up to 4 days.

12. Method according to any of the claims 6 to 10, wherein the time between step c) and step d) is up to 2 days.
